# EUROPÄISCHE PATENTANMELDUNG

(11) **EP 0 953 349 A1**
(43) Veröffentlichungstag der Anmeldung: **03.11.1999**
(21) Anmeldenummer: 99107784.3
(22) Anmeldetag: 20.04.1999
(51) Int. Cl.: A61K 31/21, A61K 47/26

(54) **Nitroglycerin und wasserfreie Lactose enthaltende Feststoffmischungen**

(30) Priorität: 29.04.1998 DE 19819012
(71) Anmelder: Dynamit Nobel GmbH, 53840 Troisdorf (DE)
(72) Erfinder: Heinzelmann, Walter Dr., 51519 Odenthal (DE); Ruloff, Cornelius Dr., 51377 Leverkusen (DE)
(74) Vertreter: Uppena, Franz, Dr.

(57) **Zusammenfassung**

Die vorliegende Erfindung betrifft feuersichere, nicht explosionsfähige, Nitroglycerin und wasserfreie Lactose enthaltende Feststoffmischungen, die gemäß ADR/RID-, IATA, IMCO- und/oder UN-Richtlinien nicht als Sprengstoff klassifiziert werden müssen, Verfahren zur Herstellung solcher Feststoffmischungen sowie die Verwendung wasserfreier Lactose zur Herstellung von feuersicheren, nicht explosionsfähigen, Nitroglycerin und Lactose enthaltenden Feststoffmischungen, die gemäß ADR/RID-, IATA, IMCO- und/oder UN-Richtlinien nicht als Sprengstoff klassifiziert werden müssen.

## Beschreibung

Gegenstand der vorliegenden Erfindung ist die Verwendung von Lactose als Excipient von Nitroglycerin.

Nitroglycerin (Glycerintrinitrat, Propantriol-1,2,3-trinitrat) ist ein wohlbekannter flüssiger Nitratester, der als Arzneiwirkstoff breite Anwendung in zahlreichen Arzneimitteln zur Koronartherapie gefunden hat, die sowohl für eine schnelle Wirkung in Notfällen als auch für eine langsame Freigabe des Wirkstoffes für einen verlängerten Schutz vor angina pectoris Attacken formuliert werden können.

Je nach der Zusammensetzung von Arzneimitteln für wichtige koronartherapeutische Einsatzgebiete, z. B. zur oralen, dermalen oder parenteralen Anwendung, stellt die Verfügbarkeit von Nitroglycerin für pharmazeutische Produktionszwecke ein ständiges Problem dar. Nitroglycerin als ein hochexplosiver Wirkstoff kann nur in Form völlig phlegmatisierter und handhabungssicherer Konzentrate oder als Endarzneimittel in bulk-Form für die Handhabung und den Transport verfügbar gemacht werden, welche nur unter Sicherheitsbedingungen in einem Sprengstoffwerk und unter Verwendung geeigneter, inerter Trägermaterialien als Excipienten hergestellt werden können, die entsprechend den speziellen physikalisch-chemischen Eigenschaffen des Nitroglycerins ausgewählt und Gegenstand von Arzneibuchmonographien sein müssen.

Unterschiedliche Typen von Arzneimitteln, die Nitroglycerin enthalten, benötigen unterschiedliche Typen von Excipienten zur Inkorporierung von Nitroglycerin, um Produkte zu erhalten, die, selbst unter extremen Bedingungen, wie z. B. unter Feuereinwirkung im Falle eines Brandes, absolut handhabungssicher während der Produktion, der Verpackung, der Lagerung, des Transportes und der Endanwendung sein müssen.

Hauptsächlich praktische Erfordernisse haben zur Entwicklung von zwei verschiedenen Gruppen von phlegmatisierten Konzentraten geführt, die bereits kommerzialisiert sind und als Vormischungen zur weiteren Verarbeitung dienen:
- Gruppe A:: Formulierung von Nitroglycerin in flüssigen Excipienten, z. B. in: Alkoholen,
mittelkettigen Triglyceriden,
pflanzlichen oder synthetischen Ölen
organischen Lösemitteln,
organischen Polymeren (Lösungen, Dispersionen).
- Gruppe B:: Formulierungen von Nitroglycerin in festen Excipienten, z. B. in: Zucker und Polysacchariden,
Cellulose und Derivaten,
polymeren Materialien,
anorganischen Excipienten.

Unter den in Gruppe B enthaltenen Materialien haben insbesondere Feststoffkonzentrate von Nitroglycerin in Zuckern, wie z. B. Lactose, kommerzielles Interesse als vielseitig verwendbare pharmazeutische Zwischenprodukte, z. B. für die Herstellung von Tabletten, Kapseln, Salben oder Pflastern, gefunden.

Maßgeblich für die Erteilung einer Transportgenehmigung durch nationale und/oder internationale Transportbehörden sind die Resultate von Sicherheitstests sowie Materialsicherheitsdaten, die nach national und/oder international eingeführten Sicherheits-Standardprüfmethoden sowie gemäß gesetzlicher Anforderungen erhalten werden, wie sie von den ADR/RID-, IATA, IMCO- und UN-Richtlinien und den diesbezüglichen Test-Manuals vorgeschrieben werden.

Entscheidend für die Klassifizierung einer Substanz als Sprengstoff oder als Nicht-Sprengstoff sind die Ergebnisse bestimmter Tests, die für die Bestimmung der nachstehend genannten Parameter durchzuführen sind:
- Thermische Empfindlichkeit unter Einschluß nach dem Koenen-Test; UN-Testhandbuch, Serie 2 (b), 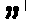 Koenen test".
- Detonationsfähigkeit nach dem 2''-Stahlrohr-Test; UN-Testhandbuch, Test A, Serie A.1, BAM 50/60 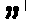 steel tube".
- Schlagempfindlichkeit nach dem Fallhammertest; UN-Testhandbuch, Test 3 (a)(i), 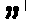 BAM Fallhammer".
- Reibempfindlichkeit nach dem BAM Friktionstest; UN-Testhandbuch, Test 3 (b)(i), 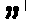 BAM friction apparatus"
- Thermische Empfindlichkeit nach dem 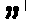 bonfire-test" unter Verwendung von Original-Lager- und Transportbehältern; UN-Testhandbuch, 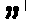 external fire (bonfire) test", Serie 6 (c)

Für jeden Test sind Grenzwerte angegeben, die von Nicht-Sprengstoffen nicht überschritten werden dürfen. Werden diese Grenzwerte aber auch nur bei einem einzigen Test überschritten, beispielsweise beim 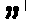 Koenen-Test" und/oder dem 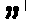 bonfire-test", ist das untersuchte Material als Sprengstoff zu klassifizieren. Nicht-Sprengstoffe dagegen unterliegen weiterhin den erforderlichen Klassifizierungen als Gefahrgut, wie z. B. in Klasse 3 (brennbar), in Klasse 4.1 (brennbare feste Stoffe) oder in Klasse 6 (giftig).

Eine typische, handelsübliche Standard-bulk-Formulierung für Nitroglycerin mit festen Excipienten stellt beispielsweise die Feststoffmischung von 10 Gew.-% Nitroglycerin (C₃H₅N₃O₉) in 90 Gew.-% Lactosemonohydrat (C₁₂H₂₂O₁₁ x 1 H₂O) dar. Auch diese Formulierung wurde mit dem obengenannten Prüfprogramm untersucht. Während der 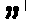 Koenen-Test", der 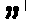 Steel tube-Test", der 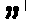 BAM Fallhammer-Test" und der 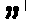 BAM friction apparatus-Test" Werte erbrachte, die unterhalb des jeweiligen Grenzwertes lagen, führte der 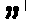 bonfire-Test" zu heftigen Explosionen. Wurden die Originalgebinde für Transport und Lagerung, in denen 50 kg (netto) der Feststoffmischung aus Nitroglycerin und Lactosemonohydrat in 100 l Fibertrommeln mit PolyethylenSackeinlagen verpackt waren, für längere Zeit offenem Feuer ausgesetzt, beobachtete man regelmäßig nach etwa 45 min. eine heftige Explosion.

Aus diesem Grund ist diese handelsübliche Standard-bulk-Formulierung von 10 Gew.-% Nitroglycerin in 90 Gew.% Lactosemonohydrat durch die nationalen und internationalen Transportbehörden als Sprengstoff in Klasse 1.1 D eingestuft worden. Produktions-, Lagerungs- und insbesondere Transport-Aktivitäten sind daher durch legislative Restriktionen so stark gehandikapt, daß das Material vom internationalen Transport praktisch ausgeschlossen ist. So besteht für dieses Material ein generelles Verbot von Lufttransporten; Straßen-, Eisenbahn- und Seetransporte dürfen nur als Sprengstofftransporte durchgeführt werden; es gelten Mengenbeschränkungen pro Einzelpackstück und Gesamtfrachtmenge; die Frachtkosten sind extrem hoch; für jeden Transport im Transitverkehr sind Einzelgenehmigungen erforderlich; je nach Empfängerland werden zusätzlich spezielle Anforderungen an Gebäude (Läger, Produktion) und die Einrichtungen zur Weiterverarbeitung gestellt; speziell geschultes und lizensiertes Personal ist notwendig; die Absatzmöglichkeiten sind stark eingeschränkt.

Auch die Zumischung geringer Mengen von Additiven, beispielsweise von feindispersem Siliziumdioxid (Aerosil), von Alkali- oder Erdalkali-carbonaten, -bicarbonaten, -hydroxiden oder -oxiden (US-A- 428378; EP-A- 0 104 877) hatten auf die Ergebnisse der relevanten Tests zur Prüfung der thermischen Empfindlichkeit beispielsweise dem Stahlblechkästchen-Test, keinen Einfluß und konnten die Explosion nicht verhindern.

Es war daher Aufgabe der vorliegenden Erfindung, eine Feststoffmischung für Nitroglycerin verfügbar zu machen, die kein Explosionsrisiko, sondern hohe thermische Stabilität aufweist und daher nicht als Sprengstoff klassifiziert zu werden braucht.

Überraschenderweise konnte eine feuersichere, nicht explosionsfähige, Nitroglycerin enthaltende Feststoffmischung durch die Verwendung wasserfreier Lactose als Excipient für Nitroglycerin bereitgestellt werden und damit die der Erfindung zugrundeliegende Aufgabe gelöst werden.

Untersuchungen der bei der Explosion im 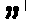 bonfire-test" ablaufenden Reaktionen durch thermogravimetrische Analyse (TGA) haben gezeigt, daß das Lactosemonohydrat, das bisher als Excipient zur Adsorption von Nitroglycerin verwendet wird, bei erhöhten Temperaturen sein Kristallwasser innerhalb eines Temperaturbereiches von 100°C bis 150°C mit anschließender Schmelze oberhalb 215°C freisetzt. Durch diesen Effekt wird das an Latosemonohydrat adsorbierte Nitroglycerin, das in Wasser praktisch unlöslich ist, durch das freigesetzte Wasser (Dampf/Kondensat) von den Kristalloberflächen des Latosemonohydrates abgewaschen und separiert. Damit ändert sich aber der Sicherheitsstatus der zuvor bei Umgebungstemperaturen völlig phlegmatisierten Feststoffmischung. Durch Migration und Anreicherung des Nitroglycerins als Flüssigphase wandelt sich die Mischung zu einem brisanten Explosivstoff, der unter den Bedingungen des 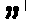 bonfire-tests" explodiert. Unterstützt und belegt wurden diese Ergebnisse auch durch die nachträglichen Untersuchungen der Explosionsstätte und der Trümmerstücke.

Die thermische Sicherheit von Feststoffgemischen, die Nitroglycerin und Lactose enthalten, kann nun überraschenderweise dadurch signifikant verbessert werden, daß anstelle von Lactosemonohydrat als Excipient wasserfreie Lactose für die Adsorption von Nitroglycerin verwendet wird.

Hergestellt werden die erfindungsgemäßen Feststoffgemische in einem kommerziell üblichen Mischer, beispielsweise in einem Planetenmischer, in dem trockene wasserfreie Lactose gemäß EP, USP, JP vorgelegt und unter Rühren reines Nitroglycerin aus einer geeigneten Dosiervorrichtung zugegeben wird. Die Mischung wird nach Ende der Zugabe noch eine Zeit lang, vorzugsweise mindestens zwei Stunden, gerührt. Die so erhaltene homogene Feststoffverreibung von Nitroglycerin in wasserfreier Lactose wird in Fibertrommeln mit Polyethylen-Sackeinlage verpackt und bei Raumtemperatur trocken gelagert.

Selbst Untersuchungen von erfindungsgemäßen Feststoffgemischen, die ≥ 10 Gew.-% Nitroglycerin mit entsprechenden Mengen an wasserfreier Lactose enthalten, ergaben in allen obengenannten Tests Werte, die deutlich unter den jeweiligen Grenzwerten lagen. Auch im Brand-Test führten die erfindungsgemäßen Feststoffgemische nicht zu Explosionen.

Versuchsreihen zur Untersuchung der thermischen Empfindlichkeit haben gezeigt, daß der verfügbare Spielraum für die thermische Sicherheit (Brandfall) eine Ausdehnung des Nitroglycerin-Anteils bis weit oberhalb 10 Gew.-% Nitroglycerin mit der entsprechenden Menge wasserfreier Lactose erlaubt.

Die erfindungsgemäßen, feuersicheren, nicht explosionsfähigen Nitroglycerin und wasserfreie Lactose enthaltenden Feststoffmischungen müssen gemäß ADR/RID, IATA, IMCO- und/oder UN-Richtlinien nicht als Sprengstoff klassifiziert werden. Sie müssen folglich als explosionssicheres Material gelten.

Die vorliegende Erfindung betrifft im einzelnen:

Feuersichere, nicht explosionsfähige, Nitroglycerin und wasserfreie Lactose enthaltende Feststoffmischungen. Diese erfindungsgemäßen Feststoffmischungen müssen gemäß ADR/RID-, IATA, IMCO- und/oder UN-Richtlinien nicht als Sprengstoff klassifiziert werden. Sie enthalten vorzugsweise eine Mischung aus (4 + x) Gew.-% Nitroglycerin und (96 - x) Gew.-% wasserfreie Lactose, bezogen auf die Mischung aus Nitroglycerin/wasserfreie Lactose, wobei x Werte von 0 bis 9, vorzugsweise die Werte 4, 6 oder 8 annehmen kann.

Darüber hinaus betrifft die Erfindung die Verwendung von wasserfreier Lactose zur Herstellung feuersicherer Nitroglycerin und Lactose enthaltenden Feststoffmischungen, insbesondere die Verwendung von (96 - x) Gew.-% wasserfreier Lactose und (4 + x) Gew.-% Nitroglycerin, bezogen auf die Mischung aus Nitroglycerin/wasserfreie Lactose, wobei x Werte von 0 bis 9, vorzugsweise die Werte 4, 6 oder 8 annehmen kann.

Weiterhin betrifft die Erfindung Verfahren zur Herstellung von feuersicheren Nitroglycerin und Lactose enthaltenden Feststoffmischungen, bei denen wasserfreie Lactose mit Nitroglycerin, insbesondere (96 - x) Gew.-% wasserfreie Lactose mit (4 + x) Gew.-% Nitroglycerin, bezogen auf die Mischung aus Nitroglycerin/wasserfreie Lactose, innig vermischt wird, wobei x Werte von 0 bis 9, vorzugsweise die Werte 4, 6 oder 8 annehmen kann.

Studien zur Bestimmung der LD 50-Werte der akuten oralen Toxizität und der akuten dermalen Toxizität von Feststoffgemischen aus 10 Gew.-% Nitroglycerin und 90 Gew.-% Lactose an Ratten ergaben jeweils Werte von >2000 mg/kg. Eine Einstufung dieses Materials in Klasse 6 (stark giftig) ist daher ebenfalls nicht erforderlich.

## Patentansprüche

1. Feuersichere, nicht explosionsfähige, Nitroglycerin und wasserfreie Lactose enthaltende Feststoffmischung.

2. Feuersichere, nicht explosionsfähige, Nitroglycerin und wasserfreie Lactose enthaltende Feststoffmischung, die gemäß ADR/RID-, IATA, IMCO- und/oder UN-Richtlinien nicht als Sprengstoff klassifiziert werden muß.

3. Feuersichere, nicht explosionsfahige, Nitroglycerin und wasserfreie Lactose enthaltende Feststoffmischung gemäß Anspruch 1 oder 2, dadurch gekennzeichnet, daß sie eine Mischung aus (4 + x) Gew.-% Nitroglycerin und (96 - x) Gew.-% wasserfreie Lactose, bezogen auf die Mischung aus Nitroglycerin/wasserfreie Lactose, enthält, wobei x Werte von 0 bis 9, vorzugsweise die Werte 4, 6 oder 8 annehmen kann.

4. Feuersichere, nicht explosionsfähige, Nitroglycerin und wasserfreie Lactose enthaltende Feststoffmischung gemäß Anspruch 1 oder 2, dadurch gekennzeichnet, daß sie 13 Gew.-% Nitroglycerin und 87 Gew.-% wasserfreie Lactose, bezogen auf die Mischung aus Nitroglycerin/wasserfreie Lactose, enthält.

5. Verwendung von wasserfreier Lactose zur Herstellung einer feuersicheren, nicht explosionsfähigen, Nitroglycerin und Lactose enthaltenden Feststoffmischung.

6. Verwendung von wasserfreier Lactose zur Herstellung einer feuersicheren, nicht explosionsfähigen, Nitroglycerin und Lactose enthaltenden Feststoffmischung, die gemäß ADR/RID-, IATA, IMCO- und/oder UN-Richtlinien nicht als Sprengstoff klassifiziert werden muß.

7. Verwendung von wasserfreier Lactose zur Herstellung einer feuersicheren, nicht explosionsfähigen, Nitroglycerin und Lactose enthaltenden Feststoffmischung gemäß Anspruch 5 oder 6, dadurch gekennzeichnet, daß (96 - x) Gew.-% wasserfreie Lactose und (4 + x) Gew.-% Nitroglycerin, bezogen auf die Mischung aus Nitroglycerin/wasserfreie Lactose, verwendet wird, wobei x Werte von 0 bis 9, vorzugsweise die Werte 4, 6 oder 8 annehmen kann.

8. Verfahren zur Herstellung einer feuersicheren, nicht explosionsfähigen, Nitroglycerin und Lactose enthaltenden Feststoffmischung, dadurch gekennzeichnet, daß wasserfreie Lactose mit Nitroglycerin innig vermischt wird.

9. Verfahren zur Herstellung einer feuersicheren, nicht explosionsfähigen, Nitroglycerin und Lactose enthaltenden Feststoffmischung, die gemäß ADR/RID-, IATA, IMCO- und/oder UN-Richtlinien nicht als Sprengstoff klassifiziert werden muß, dadurch gekennzeichnet, daß wasserfreie Lactose mit Nitroglycerin innig vermischt wird.

10. Verfahren zur Herstellung einer feuersicheren, nicht explosionsfähigen, Nitroglycerin und Lactose enthaltenden Feststoffmischung gemäß Anspruch 8 oder 9, dadurch gekennzeichnet, daß (96 - x) Gew.-% wasserfreie Lactose mit (4 + x) Gew.-% Nitroglycerin, bezogen auf die Mischung aus Nitroglycerin/wasserfreie Lactose, innig vermischt wird, wobei x Werte von 0 bis 9, vorzugsweise die Werte 4, 6 oder 8 annehmen kann.
